(19) Europäisches Patentamt
European Patent Office
Office européen des brevets

(11) Veröffentlichungsnummer: **0 406 659 A1**

(12) **EUROPÄISCHE PATENTANMELDUNG**

(21) Anmeldenummer: **90112003.0**

(22) Anmeldetag: **25.06.90**

(51) Int. Cl.5: **C07D 413/04**, C07D 211/98, A61K 31/41, //(C07D413/04, 271:00,211:00)

(30) Priorität: **03.07.89 DE 3921796**

(43) Veröffentlichungstag der Anmeldung: **09.01.91 Patentblatt 91/02**

(84) Benannte Vertragsstaaten: **AT BE CH DE DK ES FR GB IT LI NL SE**

(71) Anmelder: **CASSELLA Aktiengesellschaft**
**Hanauer Landstrasse 526**
**D-6000 Frankfurt am Main 61(DE)**

(72) Erfinder: **Kujath, Eckhard, Dr.**
**Bohnhoeffer Strasse 27**
**D-6457 Maintal 1(DE)**
Erfinder: **Baumgartner, Christian, Dr.**
**Schwilkenhofstrasse 4**
**D-7000 Stuttgart 40(DE)**
Erfinder: **Schönafinger, Karl, Dr.**
**Holunderweg 8**
**D-8755 Alzenau(DE)**
Erfinder: **Beyerle, Rudi, Dr.**
**An der Pfaffenmauer 44**
**D-6000 Frankfurt am Main 60(DE)**
Erfinder: **Just, Melitta, Dr.**
**Theodor-Heuss-Strasse 80**
**D-6070 Langen(DE)**
Erfinder: **Bohn, Helmut, Dr.**
**Kranzbergring 11**
**D-6369 Schöneck 1(DE)**
Erfinder: **Ostrowski, Jörg, Dr.**
**Eschenstrasse 20**
**D-6458 Rodenbach(DE)**

(74) Vertreter: **Urbach, Hans-Georg, Dr. et al**
**CASSELLA AKTIENGESELLSCHAFT**
**Patentabteilung Hanauer Landstrasse 526**
**D-6000 Frankfurt am Main 61(DE)**

(54) **Substituierte 3-Aminosydnonimine, Verfahren zu ihrer Herstellung und ihre Verwendung.**

(57) Substituierte 3-Aminosydnonimine der Formel I

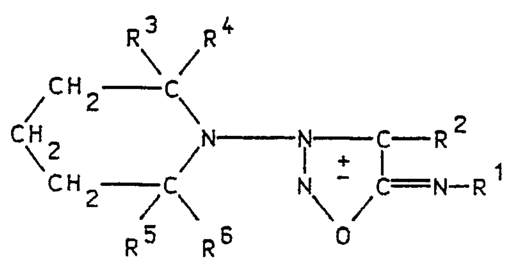

(I)

und ihre pharmakologisch annehmbaren Säureadditionssalze, worin
$R^1$ z.B. Wasserstoff;
$R^2$ z.B. einen Alkylrest;
mindestens zwei der Reste $R^3$, $R^4$, $R^5$, $R^6$ Alkylreste und die anderen Wasserstoff bedeuten, werden durch Cyclisierung einer Verbindung der Formel II

EP 0 406 659 A1

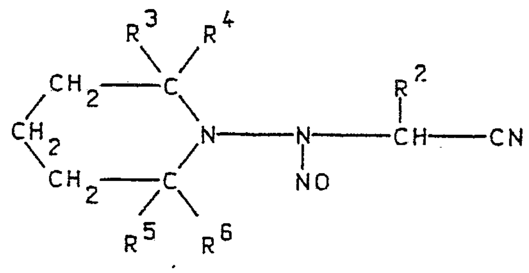

(II)

und gegebenenfalls anschließende Acylierung hergestellt und besitzen wertvolle pharmakologische Eigenschaften.

# SUBSTITUIERTE 3-AMINOSYDNONIMINE, VERFAHREN ZU IHRER HERSTELLUNG UND IHRE VERWENDUNG

Die Erfindung betrifft pharmakologisch wirksame substituierte 3-Aminosydnonimine der allgemeinen Formel I

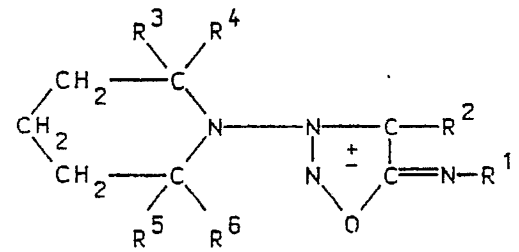

(I)

und ihre pharmakologisch annehmbaren Säureadditionssalze, worin

$R^1$ Wasserstoff oder den Rest $-COR^7$;

$R^2$ einen Alkylrest mit 1 bis 8 C-Atomen oder einen gegebenenfalls substituierten Aralkylrest mit 1 bis 4 C-Atomen im Alkylrest und 6 bis 12 Aryl-C-Atomen;

$R^3$, $R^4$, $R^5$, $R^6$ Wasserstoff oder einen Alkylrest mit 1 bis 4 C-Atomen;

$R^7$ einen aliphatischen Rest mit 1 bis 4 C-Atomen, der auch durch Alkoxy mit 1 bis 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind.

Die Erfindung betrifft ferner ein Verfahren zur Herstellung der erfindungsgemäßen Verbindungen der Formel I, die zur Herstellung der Verbindungen der Formel I benutzten N-substituierten N-Nitroso-aminoacetonitrile der allgemeinen Formel II

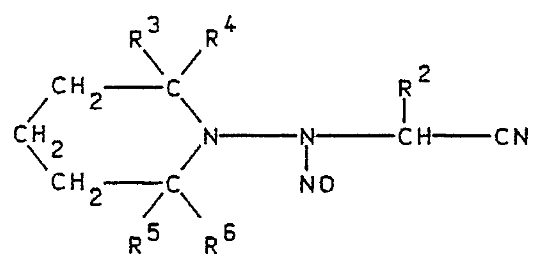

(II)

worin $R^2$, $R^3$, $R^4$, $R^5$ und $R^6$ die bereits genannten Bedeutungen besitzen, und mindestens zwei der Reste $R^3$, $R^4$, $R^5$, $R^6$ Alkylreste sind, sowie ihre Verwendung.

Aliphatische Reste, Alkylreste und Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Aralkyl oder als Alkoxycarbonyl.

Die für $R^2$ stehenden Aralkylreste können im Arylrest unsubstituiert oder durch 1 bis 3 Halogenatome und/oder 1 bis 3 Alkylreste mit 1 bis 4 C-Atomen und/oder 1 bis 3 Alkoxyreste mit 1 bis 4 C-Atomen und/oder 1 bis 2 Nitrogruppen und/oder 1 bis 2 Hydroxygruppen und/oder 1 bis 3 Alkylthioreste mit 1 bis 4 C-Atomen und/oder einen Trifluormethylrest mono-, di- oder trisubstituiert sein.

Arylreste mit 6 bis 10 C-Atomen, die auch gegebenenfalls substituiert sein können, sind z.B. Phenyl und α- oder β-Naphthyl. Arylreste mit 6 bis 12 C-Atomen, die im Zusammenhang mit einem für $R^2$ stehenden Aralkylrest auftreten und die auch substituiert sein können, sind z.B.: Phenyl, α- oder β-Naphthyl, Biphenylyl.

Vorzugsweise bedeutet $R^2$ einen Alkylrest mit 1 bis 8 C-Atomen, insbesondere mit 1 bis 6 C-Atomen, oder einen Phenylalkylrest mit 1 bis 4 C-Atomen, insbesondere 1 oder 2 C-Atomen, im Alkylrest.

Für $R^3$, $R^4$, $R^5$ und $R^6$ stehende Alkylreste können gleich oder verschieden sein. In der Regel sind sie gleich. Für $R^3$ bis $R^6$ kommen vor allem geradkettige Alkylreste in Betracht; bevorzugt sind Methylreste.

Vorzugsweise sind zwei der Reste $R^3$, $R^4$, $R^5$ und $R^6$ Alkylreste, vorzugsweise Methylreste, wobei diese beiden Alkylreste sowohl an dasselbe C-Atom des Piperidinrings (C-2 oder C-6), als auch an verschiedene C-Atome des Piperidinrings (C-2 und C-6) gebunden sein können. Besonders bevorzugt ist einer dieser beiden Alkylreste, insbesondere Methylreste, an C-2, der andere an C-6 gebunden. Diese beiden Alkylreste können sowohl cis-ständig als auch trans-ständig sein. C-2 und C-6 des Piperidinrings können gegebenenfalls unabhängig voneinander sowohl R-Konfiguration als auch S-Konfiguration aufweisen. Die Erfindung umfaßt sowohl die möglichen einzelnen Stereoisomeren der allgemeinen Formel I als auch Mischungen mehrerer Stereoisomerer der allgemeinen Formel I mit beliebiger Zusammensetzung.

Als für $R^7$ stehende aliphatische Reste kommen insbesondere Alkylreste mit 1 bis 4 C-Atomen in Betracht. Als für $R^7$ stehende aliphatische Reste, die durch Alkoxy mit 1 bis 4 C-Atomen substituiert sind, ist insbesondere der Methoxymethylrest zu nennen. Als für $R^7$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^7$ stehender bicycloaliphatischer Rest kommt insbesondere das 2,6,6-Trimethylbicyclo-(3.1.1)heptan-3-yl (=Pinanyl-3) in Betracht.

Als für $R^7$ stehender tricycloaliphatischer Rest kommt insbesondere das Tricyclo(3.3.1.1$^{3,7}$)decan-1-yl (=Adamantanyl) in Betracht. Als für $R^7$ stehende Alkoxyreste kommen insbesondere Methoxy- und Ethoxyreste in Betracht. Als für $R^7$ stehender Alkoxycarbonylrest kommt insbesondere der Ethoxycarbonylrest in Betracht. Als für $R^7$ stehende Arylreste sind z.B. $\alpha$- oder $\beta$-Naphthylreste, insbesondere aber der Phenylrest, zu nennen. Als für $R^7$ stehende Aryloxyreste sind z.B. $\alpha$- oder $\beta$-Naphthoxyreste, insbesondere aber der Phenoxyrest, zu nennen. Die für $R^7$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht. Als für $R^7$ stehende substituierte Arylreste sind insbesondere zu nennen: Methylphenyl (=Tolyl), Nitrophenyl, Chlorophenyl und Methoxyphenyl.

$R^1$ bedeutet vorzugsweise Wasserstoff, Ethoxycarbonyl oder Benzoyl. Bevorzugte Verbindungen der Formel I sind solche, die einen oder insbesondere mehrere der bevorzugten, vor allem der besonders bevorzugten Reste enthalten.

Besonders bevorzugt sind die pharmakologisch annehmbaren Säureadditionssalze, insbesondere die Hydrochloride des 4-Benzyl-3-(2,6-dimethylpiperidino)-sydnonimins und des 3-(2,6-Dimethylpiperidino)-4-(2-phenethyl)sydnonimins.

Eine Verbindung der allgemeinen Formel I kann dadurch hergestellt werden, daß eine Verbindung der allgemeinen Formel II zu einer Verbindung der allgemeinen Formel Ia

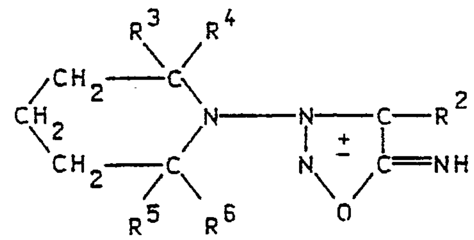

(Ia)

cyclisiert wird und daß diese oder ein Säureadditionssalz davon für den Fall, daß eine Verbindung der Formel I mit $R^1$ = $-COR^7$ hergestellt werden soll, mit einem Acylierungsmittel, das den Rest $-COR^7$ einführt, acyliert wird und die so erhaltene Verbindung gegebenenfalls in ein pharmakologisch annehmbares Säureadditionssalz überführt wird.

Die Cyclisierung der Verbindungen II zu den Verbindungen Ia wird in einem geeigneten organischen oder anorganischen Lösungs- oder Dispergiermittel unter Zusatz eines Cyclisierungsmittels, normalerweise bei Temperaturen von 0 bis 40°C, vorzugsweise bei 0 bis 20°C, durchgeführt. Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Bei der Cyclisierung wird normalerweise das entsprechende Säureadditionssalz der Verbindung Ia erhalten.

Geeignete Lösungs- oder Dispergiermittel sind z.B. Alkohole, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere

solche mit 2 bis 8 C-Atomen im Molekül, wie z.B. Diethylether, Methyl-ethyl-ether, Di-n-propyl-ether, Diisopropyl-ether, Methyl-n-butylether, Ethyl-propyl-ether, Di-butyl-ether, Tetrahydrofuran; 1,4-Dioxan, 1,2-Dimethoxyethan, Bis-β-methoxy-ethylether; Oligoethylen-glykol-dimethyl-ether, wie z.B. Pentaglyme; Carbonsäurealkylester, insbesondere solche mit 3 bis 8 C-Atomen im Molekül, wie z.B Essigsäuremethyl- oder -ethylester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Diisobutylketon, Cyclopentanon, Cyclohexanon, Benzophenon, Acetophenon; aliphatische Kohlenwasserstoffe, wie z.B. Hexan, Heptan, niedrig- und hochsiedende Petrolether; cycloaliphatische Kohlenwasserstoffe, wie z.B. Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, Chlorbenzol, Dichlorbenzol; Hexamethylphosphorsäuretriamid; Sulfoxide, wie z.B. Dimethyl-sulfoxid; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^1$ Wasserstoff bedeutet.

Die Acylierung der Verbindung der Formel Ia, die auch in Form eines Säureadditionssalzes vorliegen kann, zur Einführung des Restes $R^1$ = $-COR^7$ kann in an sich bekannter Weise mit einem geeigneten Acylierungsmittel der Formel III durchgeführt werden

$$X-\overset{\overset{\displaystyle O}{\|}}{C}-R^7 \qquad (III)$$

worin X einen nukleophil abspaltbaren Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbe sondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^7$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird in einem geeigneten Lösungs- oder Dispergiermittel oder in einem Überschuß des Acylierungsmittels, zweckmäßigerweise unter Rühren, bei Temperaturen von 0°C bis zur Siedetemperatur des Lösungs-oder Acylierungsmittels, insbesondere 0 bis 50°C, vorzugsweise von 0 bis 20°C, durchgeführt.

Bei der Acylierung der Verbindung der Formel Ia wird das Acylierungsmittel der Formel III zweckmäßigerweise in äquivalenter Menge oder in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1 : (1 bis 1,2).

Falls bei der Acylierungsreaktion eine Säure abgespalten wird, ist der Zusatz eines Säurefängers, wie z.B. eines Alkalihydroxids, wie z.B. Natrium-, Kalium-oder Lithiumhydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Verbindungen der Formel III sind Acylierungsmittel und stellen somit z.B. dar: für X = Halogen Säurehalogenide bzw. Halogenameisensäureester, von denen Säurechloride und Chlorameisensäureester bevorzugt sind; für -OH, Carbonsäuren; für -O-Alkyl und -O-Aryl Ester, von denen die Tolyl-, 2,4-Dinitro- oder 4-Nitrophenylester bevorzugt sind; für $-O-CO-R^7$ Anhydride; für -O-CO-O-Alkyl gemischte Carbonsäure-Kohlensäure-Anhydride; oder heterocyclische Amide oder Azolide. Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N′-disubstituierte Carbodiimide, insbesondere wenn sie mindestens einen sekundären oder tertiären Alkylrest enthalten, wie z.B. Diisopropyl-, Dicyclohexyl- oder N-Methyl-N′-tert.butylcarbodiimid (vgl. Methodicum Chimicum, Verlag G.Thieme, Stuttgart, Bd.6, (1974), S.682/683, und Houben-Weyl, Methoden

der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N′-Disuccinimido-carbonat, Diphthalimido-carbonat, 1,1′-(Carbonyldioxy)-dibenzotriazol oder Di-2-pyridyl-carbonat (vgl. z.B. Tetrahedron Letters, Vol.25, No. 43, 4943-4946), gegebenenfalls in Anwesenheit geeigneter Katalysatoren, wie z.B. 4-Dime thylaminopyridin. Ferner sind als Aktivierungsmittel N,N′-Carbonyldiazole, wie z.B. N,N′-Carbonyl-diimidazol, 2,2′-Carbonylditriazol(1,2,3), 1,1′-Carbonyl-ditriazol-(1,2,4), N,N′-Carbonyl-dipyrazol, 2,2′-Carbonyl-ditetrazol, N,N′-Carbonyl-benzimidazol oder N,N′-Carbonylbenztriazol, geeignet (vgl. z.B. H.A. Staab, M. Lücking und F.H. Dürr, Chem. Ber. 95 , (1962), 1275 ff, H. A. Staab und A. Mannschreck, Chem. Ber. 95 , (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Als N,N′-Carbonyl-diazol wird häufig das käufliche N,N′-Carbonyl-diimidazol verwendet. Die anderen N,N′-Carbonylazole sind aber aus dem jeweiligen Azol und Phosgen ebenfalls leicht zugänglich.

Als Aktivierungsmittel für die Carbonsäure sind ferner geeignet: Derivate der Oxalsäure, wie z.B. Oxalylchlorid (vgl. z.B. GB-PS 2 139 225) oder N,N′-Oxalyl-diazole wie z.B. 1,1′-Oxalyldi-imidazol, 1,1′-Oxalyldi-1,2,4-triazol und 1,1′-Oxalyldi-1,2,3,4-tetrazol (vgl. z.B. Shizuaka Murata, Bull.Chem. Soc.Jap. 57 , 3597-3598 (1984)); Methylethylphosphinsäureanhydrid (vgl. z.B. DE-OS 31 01 427); Diphosphortetrajodid (Chem. Lett. 1983 , 449); Dialkyldisulfit (Indian J. Chem. 21 , 259 (1982)); oder andere reaktive Agentien.

Die Acylierung der Verbindung der Formel Ia mit dem Acylierungsmittel III wird, wie bereits erwähnt, in einem geeigneten Lösungs- oder Dispergiermittel oder in einem Überschuß des Acylierungsmittels durchgeführt. Geeignete Lösungs- oder Dispergiermittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Aminosydnonimine der allgemeinen Formel I können mit anorganischen oder organischen Säuren Säureadditionssalze bilden. Pharmakologisch annehmbare Säureadditionssalze werden bevorzugt. Zur Bildung derartiger Säureadditionssalze sind anorganische oder organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden. Bei der Synthese der Verbindungen der Formel Ia fallen normalerweise die Säureadditionssalze an.

Aus den Säureadditionssalzen können die freien Verbindungen der allgemeinen Formel I gegebenenfalls in bekannter Weise, d.h. durch Auflösen oder Suspendieren in Wasser und vorsichtige Zugabe einer Base, z.B. Natronlauge, und anschließendes Isolieren, gewonnen werden.

Die als Ausgangsverbindungen benötigten N-substituierten N-Nitroso-amino-acetonitrile der allgemeinen Formel II können dadurch hergestellt werden, daß

a) eine Verbindung der allgemeinen Formel IV

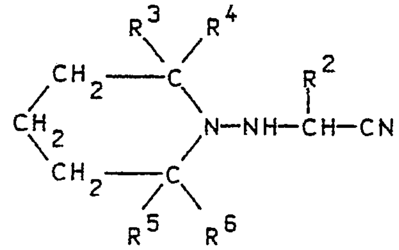

(IV)

worin $R^2$, $R^3$, $R^4$, $R^5$, $R^6$ die bereits genannten Bedeutungen besitzen, nitrosiert wird, oder daß

b) ein Säureadditionssalz einer Verbindung der Formel Ia mit einer Base umgesetzt wird.

Die Verbindungen der Formel IV können in an sich bekannter Weise nach der Streckerschen Aminonitrilsynthese aus Verbindungen der allgemeinen Formel V

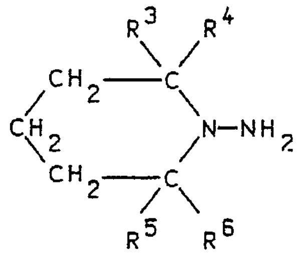

(V)

worin $R^3$, $R^4$, $R^5$ und $R^6$ die bereits genannten Bedeutungen besitzen, durch Umsetzung mit einem Aldehyd der allgemeinen Formel VI

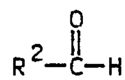

(VI)

worin $R^2$ die bereits genannte Bedeutung besitzt, und mit Blausäure bzw. einem geeigneten Cyanid, z.B. Natriumcyanid oder einem Silylcyanid, in einem geeigneten Lösungsmittel, z.B. Wasser, hergestellt werden.

Die Nitrosierung der Verbindung der Formel IV wird in bekannter Weise, zweckmäßigerweise in einem geeigneten inerten Lösungsmittel oder Lösungsmittelgemisch, vorzugsweise in Wasser, normalerweise bei Temperaturen von 0 bis 40°C und vorzugsweise bei Temperaturen von 0 bis 10°C, durchgeführt. Die Nitrosierung erfolgt z.B. mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und einer Säure, insbesondere Salzsäure, erzeugt wird. Es ist zweckmäßig, die wäßrige Lösung der Verbindung IV mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel V sind zum Teil bekannt bzw. lassen sich, ausgehend von Verbindungen der allgemeinen Formel VII

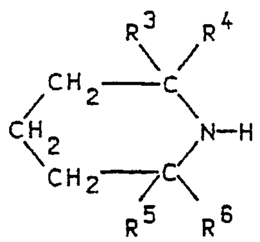

(VII)

dadurch herstellen, daß entweder

a) eine Verbindung der Formel VII zur N-Nitrosoverbindung VIIa nitrosiert und anschließend mit einem geeigneten Reduktionsmittel, beispielsweise mit Lithiumaluminiumhydrid, reduziert wird:

(VII)

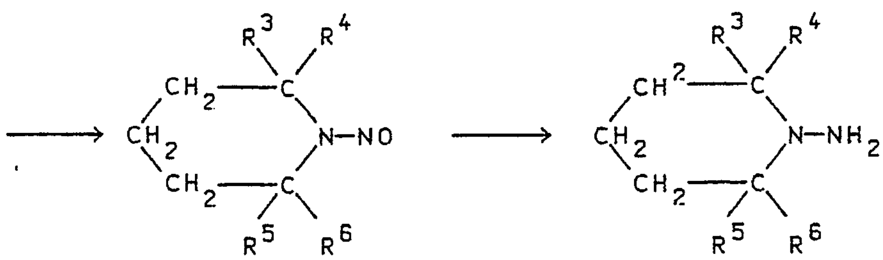

(V)

(VIIa)

oder

b) in an sich bekannter Weise eine Verbindung der Formel VII mit Kaliumcyanat im sauren Medium in das Harnstoffderivat VIII überführt wird, das dann durch Oxydation mit Natriumhypochlorit nach dem Hoffmann-Abbau in die Verbindung V überführt wird:

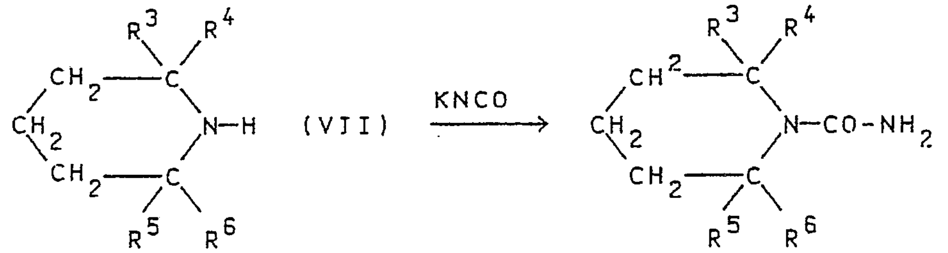


(VIII)

(VIII)

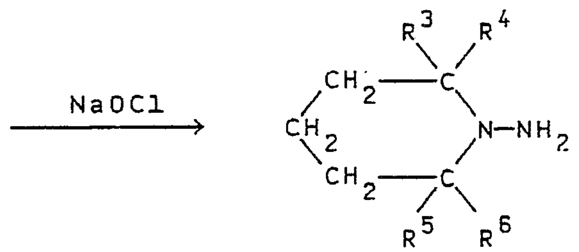


(V)

Verbindungen der Formel II können auch dadurch hergestellt werden, daß ein Säureadditionssalz einer Verbindung der Formel Ia, zweckmäßigerweise in wäßriger Lösung, mit einer Base, d.h. einer Verbindung, die in Wasser eine alkalische Reaktion einstellt, wie z.B. einem Alkalihydroxid, wie z.B. Lithium-, Natrium- oder Kalium hydroxid, einem Alkalikarbonat, wie z.B. Lithium-, Kalium- oder Natriumkarbonat oder einem Alkalibikarbonat, wie z.B. Natriumbikarbonat, oder einem Amin, insbesondere einem tertiären Amin, wie z.B. Triethylamin, behandelt wird. Die Umsetzung wird normalerweise bei 10 bis 40°C, vorzugsweise bei Raumtemperatur, durchgeführt. Es wird mindestens soviel Base zugesetzt, daß der Säurerest vollständig gebunden wird. In der Regel wird das Säureadditionssalz in Wasser oder einem Gemisch aus Wasser und einem Lösungsmittel gelöst und so viel Base zugesetzt, bis die wäßrige Lösung alkalisch reagiert. Die Bindung des Säurerestes kann auch mit einem Austauscherharz erfolgen.

Auch die Verbindungen der Formel II können mit anorganischen oder organischen Säuren Säureadditionssalze bilden, von denen pharmakologisch annehmbare Säureadditionssalze bevorzugt sind. Bezüglich der Bildung dieser Säureadditionssalze und geeigneter Säuren gilt das bereits bei den Säureadditionssalzen der Verbindungen I Gesagte.

Die Verbindungen der allgemeinen Formeln I und II und ihre pharmakologisch annehmbaren Säureadditionssalze besitzen wertvolle pharmakologische Eigenschaften. Besonders ausgeprägt ist ihre Wirkung auf das Herz-Kreislaufsystem. Verglichen mit bekannten 3-Aminosydnoniminen, in denen die 4-Position des Sydnoniminringes nur ein Wasserstoffatom trägt, weisen die Verbindungen der allgemeinen Formel I eine verlängerte Wirkdauer und/oder eine stärkere Wirkung auf. Gleiches gilt für die Verbindungen der Formel II im Vergleich mit anderen N-Nitroso-aminoacetonitrilen. Die Verbindungen der Formeln I und II und ihre pharmakologisch annehmbaren Säureadditionssalze senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formeln I und II und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I und/oder II oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze verwenden. Trägerstoffe für Weichgelatinekapseln und Suppositorien sind z.B. Fette, Wachse, halbfeste und flüssige Polyole, natürliche oder gehärtete Öle. Als Trägerstoffe für die Herstellung von Lösungen und Sirupen eignen sich z.B. Wasser, Saccharose, Invertzucker, Glukose, Polyole. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder II oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formeln I und/oder II in weiten Grenzen schwanken und z.B. 0,05 bis 50 Gew.%, vorzugsweise 0,05 bis 20 Gew.% betragen. In festen Darreichungsformen, wie Dragees, Tabletten etc. beträgt der Gehalt an einem oder mehreren Wirkstoffen der Formeln I und/oder II in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.% eines oder mehrerer Wirkstoffe der Formeln I und/oder II. Der Gehalt an einem oder mehreren Wirkstoffen der Formeln I und/oder II kann in den pharmazeutischen Präparaten teilweise, z.B. bis zu 50 Gew.%, vorzugsweise zu 5 bis 40 Gew.% durch eine oder mehrere andere therapeutisch wirksame Substanzen ersetzt sein.

Die Verbindungen der Formeln I und/oder II, ihre pharmakologisch annehmbaren Säureadditionsssalze und pharmazeutische Präparate, welche die Verbindungen der Formeln I und/oder II oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 500 mg, vorzugsweise 1 bis 100 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der Verbindungen der Formel I wurde nach einer modifizierten Methode von Godfraind and Kaba (Arch. Int. Pharmacodyn. Ther. 196 , (Suppl) 35 bis 49, 1972) und von Schüman et al (Naunyn-Schmiedeberg's Arch. Pharmacol. 289 , 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in halblogarithmisch abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind die so erhaltenen $IC_{50}$-Werte angegeben. Wie der Vergleich mit dem $IC_{50}$-Wert > $3 \cdot 10^{-4}$ für die bekannte Verbindung Molsidomin (N-Ethoxy-carbonyl-3-morpholinosydnonimin), vgl. DE-B-16 95 897, ergibt, liegen die Werte für die Verbindungen der Formel I erheblich günstiger.

TABELLE

| Verbindung (Beispiel-Nr.) | $IC_{50}$ (mol/l) |
|---|---|
| 3 | $2.4 \cdot 10^{-6}$ |
| 6 | $2.3 \cdot 10^{-6}$ |
| 8 | $2.0 \cdot 10^{-6}$ |
| Molsidomin | $>3.0 \cdot 10^{-4}$ |

Beispiel 1

3-(2,6-Dimethylpiperidino)-4-methylsydnoniminhydrochlorid

a) 2-((2,6-Dimethylpiperidino)amino)propionitril

Zur Mischung von 7,75 g 1-Amino-2,6-dimethylpiperidin, 50 ml Wasser und 5,2 ml konzentrierter Salzsäure werden unter Eiskühlung 3,9 g Natriumcyanid, gelöst in 25 ml Wasser, und 3,5 g Acetaldehyd, gelöst in 25 ml Methanol, getropft. Der pH-Wert der Lösung wird mit Salzsäure auf 7 eingestellt und das Reaktionsgemisch 2 Tage bei Raumtemperatur gerührt. Nach Abziehen des Methanols wird mit Dichlormethan extrahiert, die organische Phase mit Natriumsulfat getrocknet und eingeengt. Der verbleibende Rückstand von 9,2 g 2-((2,6-Dimethylpiperidino)amino)propionitril wird ohne weitere Reinigung weiterverarbeitet.

b) 3-(2,6-Dimethylpiperidino)-4-methylsydnoniminhydrochlorid

Zur Mischung von 8,7 g der unter a) beschriebenen Zwischenstufe, 50 ml Wasser und 4,2 ml konzentrierter Salzsäure wird unter Eiskühlung eine Lösung von 3,3 g Natriumnitrit in 20 ml Wasser getropft. Nach 1,5-stündigem Rühren bei 0°C wird der pH-Wert mit Natriumcarbonatlösung auf 5 eingestellt, die Lösung mit Ethylacetat extrahiert, die organische Phase getrocknet, teilweise eingeengt und mit 100 ml einer methanolischen Chlorwasserstofflösung versetzt. Es wird noch 2 h Chlorwasserstoff eingeleitet, der nach Stehen in der Kälte ausgefallene Niederschlag abgesaugt, das Filtrat einrotiert und der Rückstand an Kieselgel mit Dichlormethan/Methanol (9:1) chromatographiert. Es werden 6,7 g 3-(2,6-Dimethylpiperidino)-4-methylsydnoniminhydrochlorid erhalten, die nach Verrühren mit Aceton/Diethylether und Umkristallisation aus Aceton bei 127°C unter Zersetzung schmelzen.

Beispiel 2:

3-(2,6-Dimethylpiperidino)-N-ethoxycarbonyl-4-methylsydnonimin

Zu 1,3 g des unter 1) beschriebenen 3-(2,6-Dimethylpiperidino)-4-methylsydnoniminhydrochlorids in 20 ml Wasser und 10 ml Dichlormethan werden unter Eiskühlung 0,9 g Natriumhydrogencarbonat gegeben, dann wird eine Lösung von 0,6 g Chlorameisensäureethylester in 5 ml Dichlormethan zugetropft und das Reaktionsgemisch 2 h bei 0°C gerührt. Die organische Phase wird abgetrennt, die wäßrige Phase mit Dichlormethan extrahiert, die vereinigten organischen Phasen werden mit Natriumsulfat getrocknet und eingeengt. Der Rückstand wird mit Diethylether verrührt, Ungelöstes wird abgesaugt, das Filtrat einrotiert, der Rückstand mit Hexan verrieben und abgesaugt.
Ausbeute: 0,95 g 3-(2,6-Dimethylpiperidino)-N-ethoxycarbonyl-4-methylsydnonimin vom Schmelzpunkt 62-65°C.

Beispiel 3:

3-(2,6-Dimethylpiperidino)-4-hexylsydnoniminhydrochlorid

a) ((2,6-Dimethylpiperidino)amino)octansäurenitril

Zur Mischung von 26,0 g 1-Amino-2,6-dimethylpiperidin, 120 ml Wasser und 17,4 ml konzentrierter Salzsäure wird unter Eiskühlung eine Lösung von 9,95 g Natriumcyanid in 50 ml Wasser und eine Lösung von 23,1 g Heptanal in 20 ml Methanol getropft. Nach Einstellen des pH-Wertes auf 7 mit Salzsäure wird 2 Tage bei Raumtemperatur gerührt. Die Lösung wird mit Dichlormethan extrahiert, die vereinigten organischen Phasen werden mit salzsaurem Wasser vom pH 4-5 gewaschen, getrocknet und im Rotationsverdampfer eingeengt. Es werden 42,0 g 2-((2,6-Dimethyl-piperidino)amino)octansäurenitril erhalten, die ohne weitere Reinigung in der Folgestufe eingesetzt werden.

b) 3-(2,6-Dimethylpiperidino)-4-hexylsydnoniminhydrochlorid

Zur Mischung von 42,0 g der unter a) beschriebenen Aminonitrilzwischenstufe, 100 ml Wasser und 14,4 ml konzentrierter Salzsäure wird unter Eiskühlung eine Lösung von 11,5 g Natriumnitrit in 30 ml Wasser getropft. Nach 2stündigem Rühren bei 0°C wird mit Ethylacetat extrahiert, die vereinigten organischen Phasen werden getrocknet und teilweise eingeengt. Nach Zugabe von 80 ml Methanol wird 1 h bei 0°C Chlorwasserstoff eingeleitet. Der ausgefallene Niederschlag wird abgesaugt, das Filtrat einrotiert, der Rückstand an Kieselgel mit Dichlormethan/Methanol (9:1) chromatographiert und aus Ethylacetat/Acetonitril umkristallisiert.
Ausbeute: 11,8 g 3-(2,6-Dimethylpiperidino)-4-hexylsydnoniminhydrochlorid vom Schmelzpunkt 144-145°C (Zers.)

Beispiel 4:

N-Benzoyl-3-(2,6-dimethylpiperidino)-4-hexylsydnonimin

Zu 3,6 g des unter 3) beschriebenen 3-(2,6-Dimethylpiperidino)-4-hexylsydnoniminhydrochlorids in 40 ml Wasser und 20 ml Dichlormethan werden unter Eiskühlung 1,8 g Natriumhydrogencarbonat gegeben, dann wird eine Lösung von 2,0 g Benzoylchlorid in 10 ml Dichlormethan zugetropft und das Reaktionsgemisch 2 h bei 0°C gerührt. Die organische Phase wird abgetrennt, die wäßrige Phase mit Dichlormethan extrahiert, die vereinigten organischen Phasen werden getrocknet und im Rotationsverdampfer eingeengt. Der Rückstand wird an Kieselgel mit Dichlormethan und Dichlormethan/Methanol (98:2) chromatographiert. Es werden 1,4 g N-Benzoyl-3-(2,6-dimethylpiperidino)-4-hexylsydnonimin als Öl erhalten.

Beispiel 5:

3-(2,6-Dimethylpiperidino)-4-isopropylsydnoniminhydrochlorid

a) 2-((2,6-Dimethylpiperidino)amino)-3-methylbutyronitril

Zur Mischung von 6,4 g 1-Amino-2,6-dimethylpiperidin, 60 ml Wasser und 8,6 ml konzentrierter Salzsäure werden unter Eiskühlung eine Lösung von 4,9 g Natriumcyanid in 10 ml Wasser und eine Lösung von 7,2 g Isobutyraldehyd in 30 ml Methanol getropft. Nach 8tägigem Rühren bei Raumtemperatur wird das Methanol am Rotationsverdampfer abgezogen und die Lösung mit Dichlormethan extrahiert. Die vereinigten organischen Phasen werden mit essigsaurem Wasser vom pH 5 gewaschen, getrocknet und eingeengt. Es verbleiben 11,2 g 2-((2,6-Dimethylpiperidino)amino)-3-methylbutyronitril, die ohne weitere Reinigung in der

Folgestufe eingesetzt werden.

b) 3-(2,6-Dimethylpiperidino)-4-isopropylsydnoniminhydrochlorid

Zur Mischung von 10,9 g der unter a) beschriebenen Zwischenstufe, 50 ml Wasser und 5,1 ml konzentrierter Salzsäure wird unter Eiskühlung eine Lösung von4,1 g Natriumnitrit in 20 ml Wasser getropft. Nach 3stündigem Rühren wird mit Natriumhydrogencarbonatlösung pH 5 eingestellt und mit Ethylacetat extrahiert. Die vereinigten Extrakte werden getrocknet, teilweise eingeengt und mit 50 ml Methanol versetzt. Es wird 40 min Chlorwasserstoff eingeleitet. Der ausgefallene Niederschlag wird abgesaugt, das Filtrat einrotiert und der Rückstand an Kieselgel mit Dichlormethan/Methanol (9:1) chromatographiert. Nach Verrühren mit Aceton werden 1,2 g 3-(2,6-Dimethylpiperidino)-4-isopropylsydnoniminhydrochlorid abgesaugt, die nach Umkristallisation aus Aceton/Isopropanol/Diethylether ab 110°C unter Zersetzung schmelzen.

Beispiel 6:

4-Benzyl-3-(2,6-dimethylpiperidino)sydnoniminhydrochlorid

a) 2-((2,6-Dimethylpiperidino)amino)-3-phenylpropionitril

Zur Mischung von 12,8 g 1-Amino-2,6-dimethylpiperidin, 60 ml Wasser und 8,6 ml konzentrierter Salzsäure werden unter Eiskühlung 7,4 g Natriumcyanid, gelöst in 40 ml Wasser, und 18,0 g Phenylacetaldehyd, gelöst in 30 ml Methanol, getropft. Das Reaktionsgemisch wird 3 Tage bei Raumtemperatur gerührt und mit Dichlormethan extrahiert. Die vereinigten organischen Phasen werden mit essigsaurem Wasser vom pH 4-5 gewaschen, getrocknet und im Rotationsverdampfer eingeengt. Es werden 32,5 g 2-((2,6-Dimethylpiperidino)amino)-3-phenylpropionitril erhalten, die ohne weitere Reinigung weiter umgesetzt werden.

b) 4-Benzyl-3-(2,6-dimethylpiperidino)sydnoniminhydrochlorid

Zur Mischung von 32,0 g der unter a) beschriebenen Zwischenstufe, 60 ml Wasser und 10,6 ml konzentrierter Salzsäure wird unter Eiskühlung eine Lösung von 8,6 g Natriumnitrit in 20 ml Wasser getropft. Nach Zugabe von 30 ml Tetrahydrofuran wird 2 h bei 0°C gerührt und mit Ethylacetat extrahiert. Die vereinigten Extrakte werden getrocknet, teilweise eingeengt und mit 100 ml methanolischer Chlorwasserstofflösung versetzt. Es wird 2 h unter Eiskühlung Chlorwasserstoff eingeleitet. Der ausgefallene Niederschlag wird abgesaugt, das Filtrat eingeengt und an Kieselgel mit Dichlormethan/Methanol (9:1) chromatographiert. Nach Verrühren mit Isopropanol und Diethylether werden 9,2 g 4-Benzyl-3-(2,6-dimethylpiperidino)sydnoniminhydrochlorid erhalten, die nach Umkristallisation aus Aceton bei 153°C schmelzen.

Beispiel 7:

4-Benzyl-3-(2,6-dimethylpiperidino)-N-ethoxycarbonylsydnonimin

Zu 3 g des unter 6) beschriebenen 4-Benzyl-3-(2,6-dimethylpiperidino)sydnoniminhydrochlorids in 40 ml Wasser und 20 ml Dichlormethan werden unter Eiskühlung 1,6 g Natriumhydrogencarbonat gegeben, dann wird eine Lösung von 1,2 g Chlorameisensäureethylester in 10 ml Dichlormethan zugetropft und das Reaktionsgemisch 2 h bei 0°C gerührt. Die organische Phase wird abgetrennt, die wäßrige Phase mit Dichlormethan extrahiert, die vereinigten organischen Phasen werden getrocknet und im Rotationsverdampfer eingeengt. Der Rückstand kristallisiert beim Verreiben mit Diethylether.
Ausbeute: 1,4 g 4-Benzyl-3-(2,6-dimethylpiperidino)-N-ethoxycarbonylsydnonimin vom Schmelzpunkt 94-96°C.

Beispiel 8:

3-(2,6-Dimethylpiperidino)-4-(2-phenylethyl)sydnoniminhydrochlorid

a) 2-((2,6-Dimethylpiperidino)amino)-4-phenylbutyronitril

Zur Mischung von 12,8 g 1-Amino-2,6-dimethylpiperidin, 60 ml Wasser und 9,4 ml konzentrierter Salzsäure werden unter Eiskühlung eine Lösung von 5,4 g Natriumcyanid in 20 ml Wasser und eine Lösung von 14,7 g 3-Phenylpropionaldehyd in 15 ml Methanol getropft. Der pH-Wert wird mit Salzsäure auf 7 gestellt und das Reaktionsgemisch 3 Tage bei Raumtemperatur gerührt. Es wird mit Dichlormethan extrahiert, die vereinigten Extrakte werden mit salzsaurem Wasser vom pH 4 gewaschen, getrocknet und im Rotationsverdampfer eingeengt. Es werden 28,9 g 2-((2,6-Dimethylpiperidino)amino)-4-phenylbutyronitril erhalten, die ohne weitere Reinigung in die Folgestufe eingesetzt werden.

b) 3-(2,6-Dimethylpiperidino)-4-(2-phenylethyl)sydnoniminhydrochlorid

Zur Mischung von 28,6 g des unter a) beschriebenen Aminobutyronitrils, 50 ml Wasser und 8,6 ml konzentrierter Salzsäure wird unter Eiskühlung eine Lösung von 6,9 g Natriumnitrit in 20 ml Wasser getropft. Nach 2stündigem Rühren bei 0°C wird mit Ethylacetat extrahiert, die vereinigten organischen Phasen werden getrocknet, teilweise eingeengt und mit 100 ml einer methanolischen Chlorwasserstofflösung versetzt. Es wird 1 h unter Eiskühlung Chlorwasserstoff eingeleitet, die Lösung einrotiert, der Rückstand mit Diethylether und Acetonitril verrührt, der Niederschlag abgesaugt, das Filtrat einrotiert und der Rückstand an Kieselgel mit Dichlormethan/Methanol (9:1) chromatographiert. Nach Umkristallisation aus Acetonitril/Aceton werden 9,6 g 3-(2,6-Dimethylpiperidino)-4-(2-phenylethyl)sydnoniminhydrochlorid vom Schmelzpunkt 158-160°C erhalten.

Beispiel 9:

N-Benzoyl-3-(2,6-dimethylpiperidino)-4-(2-phenylethyl)sydnonimin

Zu 1,0 g des unter 8) beschriebenen 3-(2,6-Dimethylpiperidino)-4-(2-phenylethyl)-sydnoniminhydrochlorids in 30 ml Wasser und 15 ml Dichlormethan werden unter Eiskühlung 1,6 g Natriumhydrogencarbonat gegeben, dann wird eine Lösung von 1,4 g Benzoylchlorid in 5 ml Dichlormethan zugetropft. Es wird 2 h bei 0°C gerührt, die organische Phase abgetrennt, die wäßrige Phase mit Dichlormethan extrahiert, die vereinigten organischen Phasen werden getrocknet und im Rotationsverdampfer eingeengt. Umkristallisation des Rückstandes aus Hexan liefert 2,6 g N-Benzoyl-3-(2,6-dimethylpiperidino)-4-(2-phenylethyl)sydnonimin vom Schmelzpunkt 97-98°C.

In den nachfolgenden Beispielen A bis F werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 2 mg |
| Lactat (feingemahlen) | 2 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 311 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

**Ansprüche**

1. Substituierte 3-Aminosydnonimine der allgemeinen Formel I

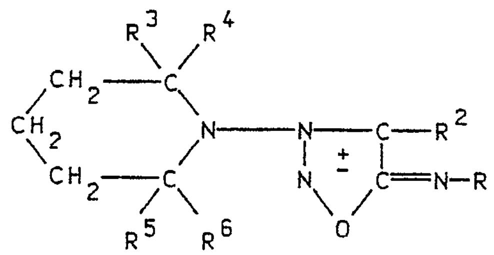

(I)

und ihre pharmakologisch annehmbaren Säureadditionssalze, worin
$R^1$ Wasserstoff oder den Rest $-COR^7$;
$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;
$R^3$, $R^4$, $R^5$, $R^6$ Wasserstoff oder einen Alkylrest mit 1 - 4 C-Atomen;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind.

2. Substituierte 3-Aminosydnonimine nach Anspruch 1, dadurch gekennzeichnet, daß $R^2$ einen Alkylrest mit 1 bis 8 C-Atomen, vorzugsweise 1 bis 6 C-Atomen, oder einen Phenalkylrest mit 1 bis 4 C-Atomen, vorzugsweise 1 oder 2 C-Atomen, im Alkylrest bedeutet.

3. Substituierte 3-Aminosydnonimine nach Anspruch 1 und/oder 2, dadurch gekennzeichnet, daß zwei der Reste $R^3$, $R^4$, $R^5$, $R^6$ Alkylreste, insbesondere daß einer der Reste $R^3$ oder $R^4$ und einer der Reste $R^5$ oder $R^6$ Alkylreste, vorzugsweise Methylgruppen, sind.

4. 4-Benzyl-3-(2,6-dimethylpiperidino)sydnonimin und seine pharmakologisch annehmbaren Säureadditionssalze, insbesondere sein Hydrochlorid.

5. 3-(2,6-Dimethylpiperidino)-4-(2-phenethyl)sydnonimin und seine pharmakologisch annehmbaren Säureadditionssalze, insbesondere sein Hydrochlorid.

6. Verfahren zur Herstellung der in einem oder mehreren der Ansprüche 1 bis 5 angegebenen Verbindungen der Formel I, dadurch gekennzeichnet, daß eine Verbindung der Formel II

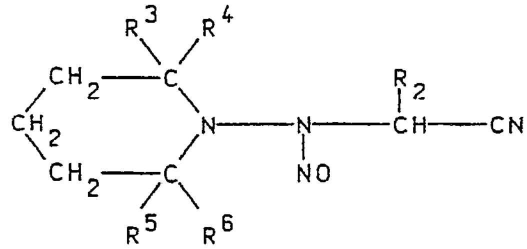

(II)

worin $R^2$, $R^3$, $R^4$, $R^5$ und $R^6$ die in einem oder mehreren der Ansprüche 1 bis 5 angegebenen Bedeutungen besitzen, zu einer Verbindung der Formel Ia

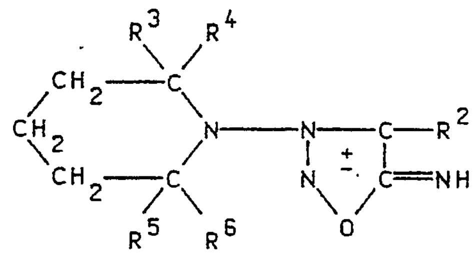

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^1$ = $-COR^7$ hergestellt wird, die Verbindung der Formel Ia oder ein Säureadditionssalz davon mit einem Acylierungsmittel acyliert wird, das den Rest $-COR^7$ einführt und die erhaltene Verbindung gegebenenfalls in ein Säureadditionssalz überführt wird.

7. Verfahren nach Anspruch 6, dadurch gekennzeichnet, daß die Cyclisierung in einem Lösungs- oder Dispergiermittel bei Temperaturen von 0 bis 40°C, vorzugsweise 0 bis 20°C, mit Hilfe von Cyclisierungsmitteln, die in wäßriger Lösung einen pH-Wert unter 3 einstellen, durchgeführt wird.

8. N-Substituierte N-Nitroso-aminoacetonitrile der allgemeinen Formel II

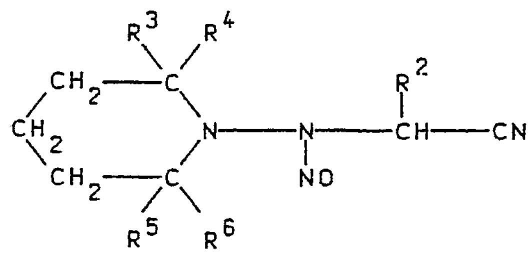

(II)

und ihre pharmakologisch annehmbaren Säureadditionssalze, worin

$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;

$R^3$, $R^4$, $R^5$, $R^6$ Wasserstoff oder einen Alkylrest mit 1 - 4 C-Atomen;

$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind.

9. Verfahren zur Herstellung der in Anspruch 8 angegebenen Verbindungen der Formel II, dadurch gekennzeichnet, daß

a) eine Verbindung der allgemeinen Formel IV

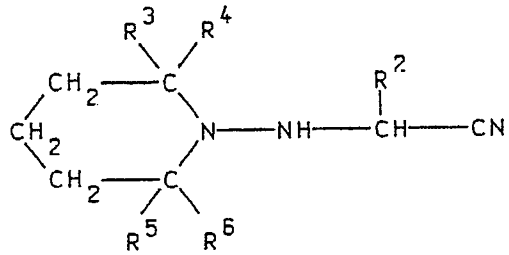

( IV )

worin $R^2$, $R^3$, $R^4$, $R^5$ und $R^6$ die in Anspruch 8 genannten Bedeutungen besitzen, nitrosiert wird, oder daß b) ein Säureadditionssalz eines substituierten 3-Aminosydnonimins der Formel Ia

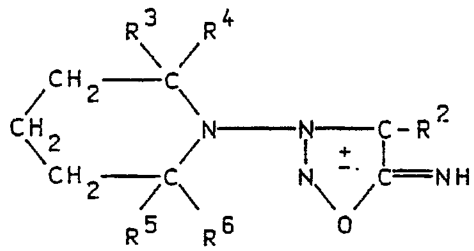

( Ia )

worin $R^2$, $R^3$, $R^4$, $R^5$ und $R^6$ die in Anspruch 8 genannten Bedeutungen besitzen, mit einer Base umgesetzt wird.

10. Verwendung eines oder mehrerer substituierter 3-Aminosydnonimine und/oder eines oder mehrerer ihrer pharmakologisch annehmbaren Säureadditionssalze der Ansprüche 1 bis 5 und/oder eines oder mehrerer N-substituierter N-Nitroso-aminoacetonitrile und/oder eines oder mehrerer ihrer pharmakologisch annehmbaren Säureadditionssalze des Anspruchs 8 als pharmakologische Wirkstoffe zusammen mit einem oder mehreren pharmakologisch annehmbaren Träger-und/oder Zusatzstoffen und gegebenenfalls noch einem oder mehreren anderen therapeutisch wirksamen Stoffen zur Herstellung pharmazeutischer Präparate.

11. Pharmazeutisches Präparat, dadurch gekennzeichnet, daß es ein oder mehrere substituierte 3-Aminosydnonimine der Ansprüche 1 bis 5 und/oder ein oder mehrere pharmakologisch annehmbare Säureadditionssalze davon und/oder ein oder mehrere N-substituierte N-Nitroso-aminoacetonitrile des Anspruchs 8 und/oder ein oder mehrere pharmakologisch annehmbare Säureadditionssalze davon als Wirkstoff oder Wirkstoffe zusammen mit einem oder mehreren pharmakologisch annehmbaren Träger- und/oder Zusatzstoffen und gegebenenfalls noch einen oder mehrere andere therapeutisch wirksame Stoffe enthält.

Patentansprüche für folgenden Vertragsstaat: ES

1. Verfahren zur Herstellung substituierter 3-Aminosydnonimine der allgemeinen Formel I

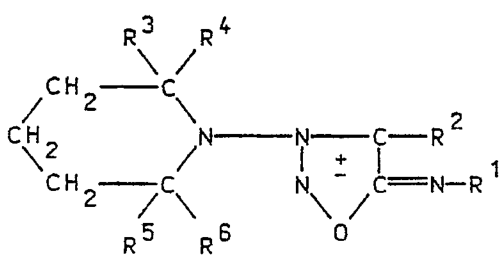

( I )

und ihrer pharmakologisch annehmbaren Säureadditionssalze, worin
$R^1$ Wasserstoff oder den Rest $-COR^7$;
$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;
$R^3$, $R^4$, $R^5$, $R^6$ Wasserstoff oder einen Alkylrest mit 1 - 4 C-Atomen;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14

C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind, dadurch gekennzeichnet, daß eine Verbindung der Formel II

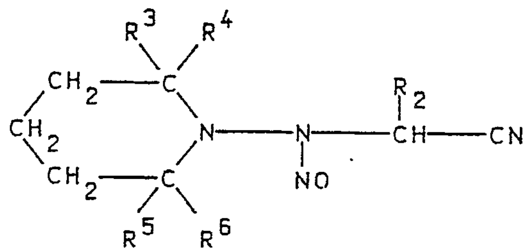

(II)

worin $R^2$, $R^3$, $R^4$, $R^5$ und $R^6$ die bereits angegebenen Bedeutungen besitzen, zu einer Verbindung der Formel Ia

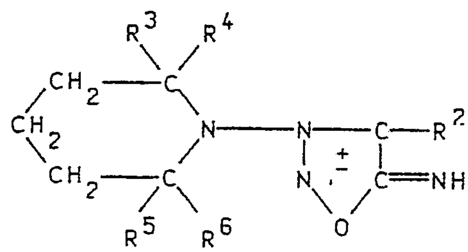

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^1$ = $-COR^7$ hergestellt wird, die Verbindung der Formel Ia oder ein Säureadditionssalz davon mit einem Acylierungsmittel acyliert wird, das den Rest $-COR^7$ einführt und die erhaltene Verbindung gegebenenfalls in ein Säureadditionssalz überführt wird.

2. Verfahren nach Anspruch 1, dadurch gekennzeichnet, daß die Cyclisierung in einem Lösungs- oder Dispergiermittel bei Temperaturen von 0 bis 40°C, vorzugsweise 0 bis 20°C, mit Hilfe von Cyclisierungsmitteln, die in wäßriger Lösung einen pH-Wert unter 3 einstellen, durchgeführt wird.

3. Verfahren nach Anspruch 1 und/oder 2, dadurch gekennzeichnet, daß eine Verbindung der Formel I eingesetzt wird, bei der $R^2$ einen Alkylrest mit 1 bis 8 C-Atomen, vorzugsweise 1 bis 6 C-Atomen, oder einen Phenalkylrest mit 1 bis 4 C-Atomen, vorzugsweise 1 oder 2 C-Atomen, im Alkylrest bedeutet.

4. Verfahren nach einem oder mehreren der Ansprüche 1 bis 3, dadurch gekennzeichnet, daß eine Verbindung der Formel I eingesetzt wird, bei der zwei der Reste $R^3$, $R^4$, $R^5$, $R^6$ Alkylreste, insbesondere daß einer der Reste $R^3$ oder $R^4$ und einer der Reste $R^5$ oder $R^6$ Alkylreste, vorzugsweise Methylgruppen, sind.

5. Verfahren nach einem oder mehreren der Ansprüche 1 bis 4, dadurch gekennzeichnet, daß die Ausgangsverbindungen so ausgewählt werden, daß das 4-Benzyl-3-(2,6-dimethylpiperidino)sydnonimin oder ein pharmakologisch annehmbares Säureadditionssalz davon, insbesondere sein Hydrochlorid, entsteht.

6. Verfahren nach einem oder mehreren der Ansprüche 1 bis 4, dadurch gekennzeichnet, daß die Ausgangsverbindungen so ausgewählt werden, daß das 3-(2,6-Dimethylpiperidino)-4-(2-phenethyl)-sydnonimin oder ein pharmakologisch annehmbares Säureadditionssalz davon, insbesondere sein Hydrochlorid, entsteht.

7. Verfahren zur Herstellung N-substituierter N-Nitroso-aminoacetonitrile der allgemeinen Formel II

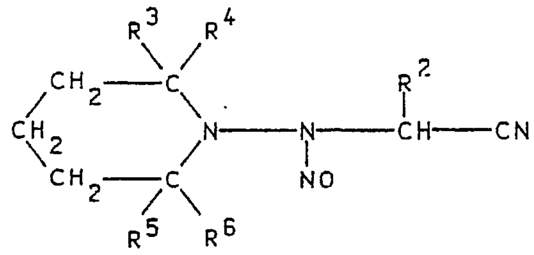

(II)

und ihrer pharmakologisch annehmbaren Säureadditionssalze, worin

$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;

$R^3$, $R^4$, $R^5$, $R^6$ Wasserstoff oder einen Alkylrest mit 1 - 4 C-Atomen;

$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind, dadurch gekennzeichnet, daß

a) eine Verbindung der allgemeinen Formel IV

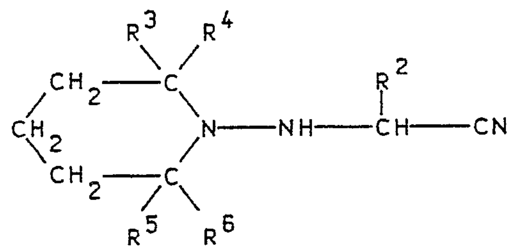

(IV)

worin $R^2$, $R^3$, $R^4$, $R^5$ und $R^6$ die genannten Bedeutungen besitzen, nitrosiert wird, oder daß

b) ein Säureadditionssalz eines substituierten 3-Aminosydnonimins der Formel Ia

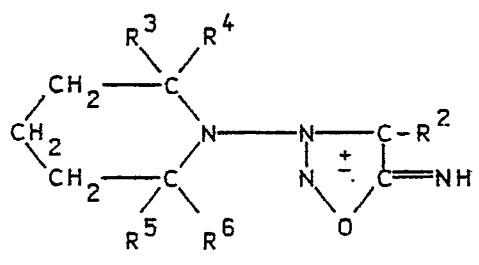

(Ia)

worin $R^2$, $R^3$, $R^4$, $R^5$ und $R^6$ die genannten Bedeutungen besitzen, mit einer Base umgesetzt wird.

8. Verwendung eines oder mehrerer substituierter 3-Aminosydnonimine der allgemeinen Formel I

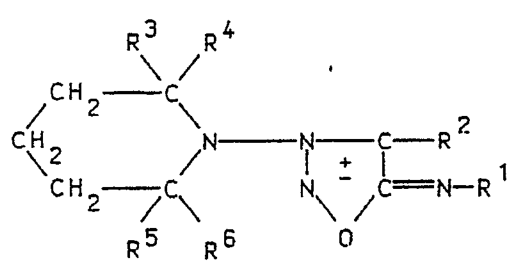

(I)

und/oder eines oder mehrerer ihrer pharmakologisch annehmbaren Säureadditionssalze, worin
$R^1$ Wasserstoff oder den Rest $-COR^7$;
$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;
$R^3$, $R^4$, $R^5$, $R^6$ Wasserstoff oder einen Alkylrest mit 1 - 4 C-Atomen;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind und/oder eines oder mehrerer N-substituierter N-Nitroso-aminoacetonitrile der allgemeinen Formel II

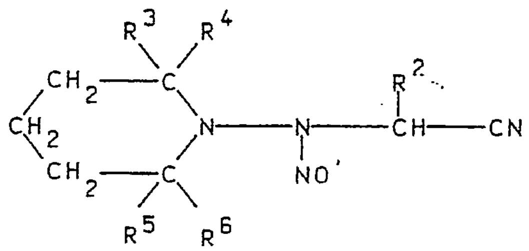

(II)

und/oder eines oder mehrerer ihrer pharmakologisch annehmbaren Säureadditionssalze, worin
$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;
$R^3$, $R^4$, $R^5$, $R^6$ Wasserstoff oder einen Alkylrest mit 1 - 4 C-Atomen;
$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind, als pharmakologischer Wirk stoff oder Wirkstoffe zusammen mit einem oder mehreren pharmakologisch annehmbaren Träger-und/oder Zusatzstoffen und gegebenenfalls noch einem oder mehreren anderen therapeutisch wirksamen Stoffen zur Herstellung pharmazeutischer Präparate.

9. Verfahren zur Herstellung eines pharmazeutischen Präparates, dadurch gekennzeichnet, daß ein oder mehrere substituierte 3-Aminosydnonimine der allgemeinen Formel I

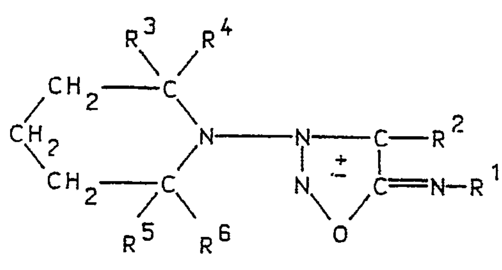

(I)

und/oder ein oder mehrere ihrer pharmakologisch annehmbaren Säureadditionssalze, worin
$R^1$ Wasserstoff oder den Rest $-COR^7$;
$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;
$R^3$, $R^4$, $R^5$, $R^6$ Wasserstoff oder einen Alkylrest mit 1 - 4 C-Atomen;

$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind, und/oder ein oder mehrere N-substituierte N-Nitroso-aminoacetonitrile der allgemeinen Formel II

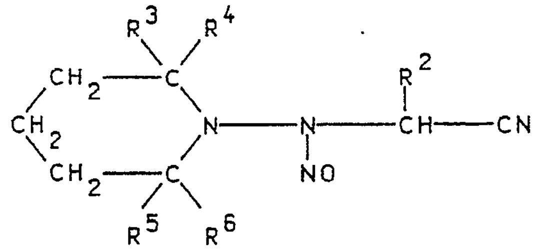

(II)

und/oder ein oder mehrere ihrer pharmakologisch annehmbaren Säureadditionssalze, worin

$R^2$ einen Alkylrest mit 1 - 8 C-Atomen oder einen gegebenenfalls substituierten Aralkylrest mit 1 - 4 C-Atomen im Alkylrest und 6 - 12 Aryl-C-Atomen;

$R^3$, $R^4$, $R^5$, $R^6$ Wasserstoff oder einen Alkylrest mit 1 - 4 C-Atomen;

$R^7$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen bedeuten, wobei mindestens zwei der Reste $R^3$, $R^4$, $R^5$, $R^6$ Alkylreste sind, als Wirkstoff oder Wirkstoffe zusammen mit einem oder mehreren pharmazeutisch annehmbaren Träger- und/oder Zusatzstoffen und gegebenenfalls noch einem oder mehreren anderen pharmakologischen Wirkstoffen in an sich bekannter Weise in eine zur Verabreichung geeignete pharmazeutische Zubereitung überführt werden.

Europäisches Patentamt

# EUROPÄISCHER RECHERCHENBERICHT

Nummer der Anmeldung

EP 90 11 2003

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 312 773 (CASSELLA AG)<br>* Ansprüche 1,7-10 *<br>– – – | 1,6,7,10, 11 | C 07 D 413/04<br>C 07 D 211/98<br>A 61 K 31/41 //<br>(C 07 D 413/04<br>C 07 D 271:00<br>C 07 D 211:00 ) |
| A | EP-A-0 023 343 (CASSELLA AG)<br>* Ansprüche 1,11,13-16 *<br>– – – | 1,6,8-11 | |
| A,D | DE-A-1 695 897 (TAKEDA CHEMICAL INDUSTRIES, LTD.)<br>* Ansprüche 1-3,6 *<br>– – – | 1,6,10,11 | |
| A | DE-A-1 670 127 (C.H. BOEHRINGER SOHN)<br>* Ansprüche 1,3,8 *<br>– – – | 1,6,10,11 | |
| A | EP-A-0 210 474 (CASSELLA AG)<br>* Ansprüche 1,9,10 *<br>– – – | 1,10,11 | |
| A,P | EP-A-0 324 408 (CASSELLA AG)<br>* Ansprüche 1,6-8 *<br>– – – | 8-11 | |
| A | DE-A-2 131 826 (SANDOZ AG)<br>* Ansprüche 1-3; Seiten 1-6 *<br>– – – | 8-11 | **RECHERCHIERTE SACHGEBIETE (Int. Cl.5)** |
| A | DE-A-2 126 035 (SANDOZ AG)<br>* Seite 4, Zeile 9 - Seite 8, Zeile 12 *<br>– – – – – | 8,10,11 | C 07 D 211/00<br>C 07 D 271/00<br>C 07 D 295/00<br>C 07 D 413/00 |

Der vorliegende Recherchenbericht wurde für alle Patentansprüche erstellt

| Recherchenort | Abschlußdatum der Recherche | Prüfer |
|---|---|---|
| Berlin | 12 Oktober 90 | HASS C V F |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument